# EUROPEAN PATENT SPECIFICATION

(11) **EP 3 064 226 B1**
(45) Date of publication and mention of the grant of the patent: **28.02.2018**
(21) Application number: 16159002.1
(22) Date of filing: 07.03.2016
(51) Int. Cl.: A61L 2/24, A47L 15/30, A47L 15/42, A47L 15/46, B08B 3/06

(54) **MACHINE AND METHOD FOR WASHING AND/OR STERILIZING LOOSE PRODUCTS**
VORRICHTUNG UND METHODE ZUM STERILISIEREN UND/ODER WASCHEN VON LOSEN PRODUKTEN
MACHINE ET PROCÉDÉ DE LAVER ET/OU STERILISATION DE PRODUITS EN VRAC

(30) Priority: 05.03.2015 IT UD20150026
(43) Date of publication of application: 07.09.2016
(73) Proprietor: Icos Pharma S.p.A., 33080 Zoppola (IT)
(72) Inventor: Zardini, Fabio, 31033 Castelfranco Veneto (TV) (IT); Casonato, Ottorino, 31033 Castelfranco Veneto (TV) (IT); Capovilla, Ivone, 31037 Loria (TV) (IT)
(74) Representative: Petraz, Davide Luigi

(56) References cited:
- DE-A1- 19 506 137
- JP-A- 2004 066 212

## Description

### FIELD OF THE INVENTION

Embodiments described here concern a machine for washing and/or sterilizing loose products and a corresponding method usable, for example, in the pharmaceutical, hospital or food sectors, or in the industrial sector for the production of loose products.

In particular, embodiments described here concern a washing and/or sterilizing machine and a corresponding method able to carry out washing and/or sterilization of the loose products in a rotating drum, which products can be, for example, stoppers for test tubes used in laboratory analyses, stoppers for phials of medicine, or small instruments or objects used in operating theatres or laboratories.

### BACKGROUND OF THE INVENTION

It is known, in the pharmaceutical or hospital fields, or in other fields, for example food, that sterile products must be used in order to prevent contamination or alterations in the substances with which they come into contact, or to prevent the introduction into places where a total absence thereof is required, of bacteria, germs, other corpuscles, particles or other potentially pathogenic or harmful agents.

Among the sterile products cited above, in all the fields mentioned, there are normally loose products, even small, such as for example stoppers for closing test tubes intended to contain substances to be subjected to laboratory analyses, stoppers for phials of drugs or generally for containers for medicines, or small instruments or objects used in operating rooms or laboratories. These can be made for example of rubber, plastic, polymer materials, metals or metal alloys.

Such loose products are sterile when they are produced and sold in packages which are also sterile, but if they are re-usable they must be washed and/or sterilized before re-use, thus extending their working life.

It is known to wash and/or sterilize loose products in washing and/or sterilizing machines provided with a sealed chamber in which a rotating drum receives the products to be treated on a loading side, or "dirty" side, washes and/or sterilizes them during a treatment cycle (normally comprised between 1 and 5 hours), and then unloads them on a so-called "sterile" unloading side, where they are generally packaged in sterile packages.

The rotating drums of such machines are partitioned into angular compartments, typically from four to twenty four, each able to contain, in every operating cycle, a desired quantity of loose products to be treated.

It is known that the loading side and the unloading side of such machines are provided with apertures through which to insert, or vice versa to remove, the loose products, respectively to be washed and/or sterilized or after they have been washed and/or sterilized, into/from the angular compartments of the rotating drum.

An example of a known machine is described in the application for a patent of industrial invention n. UD2014A000060, filed in Italy on 04.04.2014 in the name of the present Applicant.

One disadvantage in the state of the art can be that, after a complete treatment cycle, but even a partial one, the reciprocal angular position of the angular compartments of the rotating drum and the apertures for loading and unloading the loose products can be incorrect, for example misaligned.

This misalignment requires the manual intervention of an operator to return the rotating drum to a suitable angular position to perform the necessary loading or unloading steps, and inevitably causes operating slowdowns, downtimes, lost productivity and the need to dedicate a specific operator to perform the manual realignment.

Furthermore, if the machine is provided with automated mechanisms for opening and closing the angular compartments, a condition of non-alignment can lead to mechanical interferences between the moving parts, with a consequent blockage of the machine or possibly breakage of the components or mechanisms of the machine.

Another disadvantage can occur when the washing and/or sterilizing machine is to be partly loaded, that is, when it is not necessary to fill all the angular compartments with loose products. In this case, at the end of the treatment cycle, it is not possible to know if the angular compartment that is positioned in correspondence with the exit aperture is full or empty. In this eventuality, it is necessary to verify manually and visually the various angular compartments, opening them in succession until the first full first angular compartment is identified. If the first angular compartment that is verified were to be full, it would in any case be necessary to open all the successive compartments until a full round is complete, taking too much time to empty the loose products from the angular compartments.

Document JP 2004 066212 describes an apparatus for washing and drying a container made of resin for housing electronic parts and precision machine parts.

There is therefore a need to perfect a machine for washing and/or sterilizing loose products and a corresponding method that can overcome at least one of the disadvantages of the state of the art.

In particular, one purpose of the present invention is to obtain a machine for washing and/or sterilizing loose products and a corresponding method to obtain the washing and/or sterilizing of loose products which is reliable and efficient, automatically providing a precise alignment of the loading and unloading apertures and the angular compartments of the rotating drum.

Another purpose of the present invention is to obtain a machine for washing and/or sterilizing loose products and a corresponding method that are simple, quick and precise in performing the steps of loading and unloading the washed and/or sterilized loose products.

Another purpose of the present invention is to obtain a machine for washing and/or sterilizing loose products and a corresponding method that are practical and economical.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with embodiments described here, a machine is provided for washing and/or sterilizing loose products. According to the present invention the machine includes a treatment chamber provided with at least one functional loading and/or unloading aperture and containing a rotating drum provided with a plurality of angular compartments. According to one embodiment, the machine comprises at least one drum actuation element, provided with a drive shaft, configured to make the rotating drum rotate around an axis of rotation. Moreover, for each of the angular compartments, at least one corresponding reference position of the rotating drum is predefined with respect to the position of the individual compartment relative to the at least one functional loading and/or unloading aperture.

The machine also comprises at least one automatic angular detection unit configured to supply a reference signal relating to the reference angular position of each angular compartment of the rotating drum in relation to the corresponding reference position of the rotating drum.

Moreover, the machine comprises at least one control unit configured to:
i) communicate with the automatic angular detection unit;
ii) select a reference position from one of the corresponding reference positions of the rotating drum;
iii) make the rotating drum rotate to search for the selected reference position;
iv) dispose and maintain the rotating drum in the predefined reference position.

In accordance with other embodiments described here, a method is provided for washing and/or sterilizing loose products using a washing and/or sterilizing machine as described here. According to one embodiment, the method includes:
- loading angular compartments of a rotating drum through a manual functional loading aperture and/or an automatic loading aperture, with loose products to be washed and/or sterilized;
- activating the rotation of the rotating drum, proceeding to the washing and/or sterilizing of the loose products contained in respective angular compartments of the rotating drum;
- searching for and maintaining a predefined angular reference position of the rotating drum by means of an automatic angular detection unit that supplies a reference signal to a control unit relating to an angular position of a predefined angular compartment of the rotating drum in relation to the position of the manual functional loading aperture and/or the automatic loading aperture and/or an unloading aperture of the loose products;
- unloading the washed and/or sterilized loose products from the angular compartments of the rotating drum through the unloading aperture, making the rotating drum rotate step-wise with sequential angular rotation steps correlated to the angular amplitude of the angular compartments.

In accordance with other embodiments described here, a machine for washing and/or sterilizing loose products is provided. According to one embodiment, the machine includes a treatment chamber provided with at least one functional loading and/or unloading aperture and containing a rotating drum provided with a plurality of angular compartments. In accordance with possible embodiments, the machine comprises:
- at least one drum actuation element, provided with a drive shaft, configured to make the rotating drum rotate around an axis of rotation;
- at least one automatic angular detection unit configured to supply a reference signal relating to a predefined reference angular position of a predefined angular compartment of the rotating drum in relation to the position of the functional loading and/or unloading aperture, said angular position of a predefined angular compartment of the rotating drum being associated with a predefined reference position of the rotating drum;
- at least one control unit configured to communicate with the automatic angular detection unit and to command the drum actuation element, so as to make the drum rotate to search for the predefined reference position of the rotating drum and dispose the rotating drum in said predefined reference position.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a lateral view of a washing and/or sterilizing machine in accordance with embodiments described here;
- fig. 2 is a lateral cross section of a washing and/or sterilizing machine in accordance with embodiments described here;
- fig. 3 is a partly sectioned front view of a washing and/or sterilizing machine in an operating condition, in accordance with embodiments described here in a first operating condition;
- fig. 4 is an enlarged detail of fig. 3;
- fig. 5 is a partly sectioned front view of a washing and/or sterilizing machine in another operating condition, in accordance with embodiments described here in a second operating condition;
- fig. 6 is an enlarged detail of fig. 5,
- fig. 7 is a three dimensional view in separate parts of a component of the machine in fig. 2;
- fig. 8 is a partial lateral view of the component in fig. 7, in two operating positions;
- fig. 9 is a front view of the component in fig. 7;
- fig. 10 is a cross section of a detail of fig. 9.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF EMBODIMENTS

We shall now refer in detail to the various forms of embodiment of the present invention, of which one or more examples are shown in the attached drawings. Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

Before describing these forms of embodiment, we must also clarify that the present description is not limited in its application to details of the construction and disposition of the components as described in the following description using the attached drawings. The present description can provide other forms of embodiment and can be obtained or executed in various other ways. We must also clarify that the phraseology and terminology used here is for the purposes of description only, and cannot be considered as limitative. The use of terms such as "including", "comprising", "having" and their variations is intended to include the elements listed after them and their equivalents, and also additional elements. Unless otherwise specified, terms such as "mounted", "connected", "supported" and "coupled" and their variations are used in the widest sense and include both direct and indirect assemblies, connections, supports and couplings. Furthermore, the terms "connected" and "coupled" cannot be limited to physical or mechanical connections or couplings.

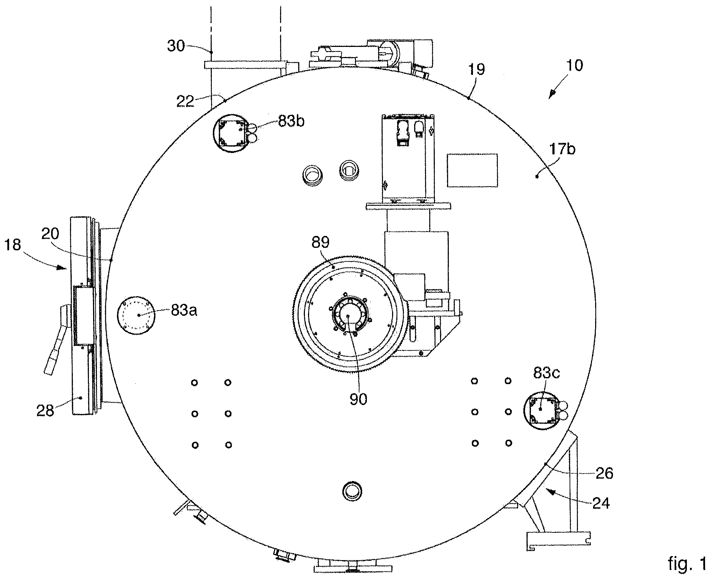

Figs. 1 to 6 are used to describe example embodiments of a washing and/or sterilizing machine 10 according to the present description, which can be used for washing and/or sterilizing loose products, small or medium size, such as for example small instruments for clinical, hospital, pharmaceutical or food use, or in general for use in the laboratory, or stoppers for test tubes or phials of drugs. The machine 10 can carry out only washing, or only sterilizing, or it can effect a combined treatment, or combined cycle of washing and sterilization.

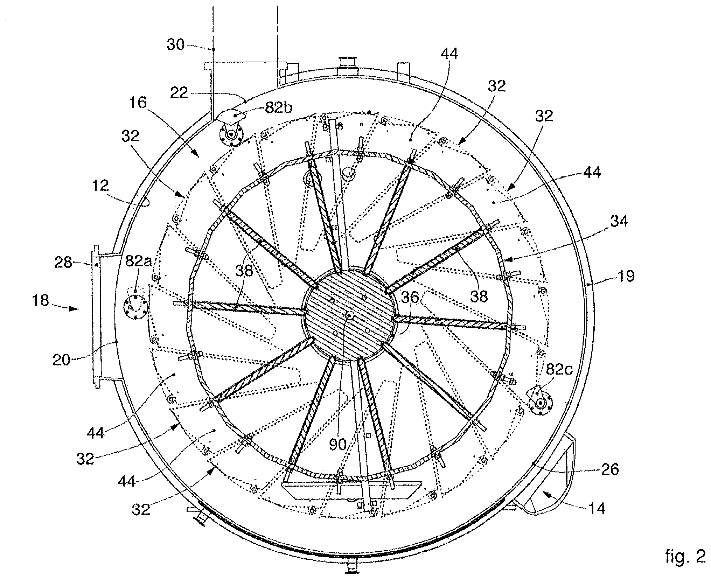

According to embodiments described using figs. 1 and 2, and combinable with all the embodiments described here, a machine 10 is provided for washing and/or sterilizing loose products which includes a treatment chamber 12. By the word "treatment" here, we mean a washing operation, or a sterilization operation, or a combined operation of washing and sterilization (combined treatment cycle or combined cycle). Therefore, in the treatment chamber 12 the loose products can be washed and/or sterilized.

The treatment chamber 12 contains a rotating drum 16 divided into angular compartments, or angular containing sectors 32, and is provided with at least one functional loading aperture 20, 22 and/or unloading aperture 26 for the loose products, for example for loading the loose products to be washed and/or sterilized from the front or top, manually or automatically, and/or to unload the washed and/or sterilized loose products. In particular, the angular compartments 32 can divide the rotating drum 16 into a plurality of angular portions, preferably all of the same size.

The machine 10 also includes a drum actuation element 85, provided with a drive shaft 90, configured to make the rotating drum 16 rotate around an axis of rotation X. The rotation can be continuous or step-wise, or intermittent, according to specific operating needs.

According to some embodiments, for at least one or for each of the angular compartments 32, at least one corresponding reference position or "zero" position of the rotating drum 16 can be defined with respect to the position of the individual compartment relating to the at least one functional loading 20, 22 and/or unloading 26 aperture.

In possible embodiments, since the geometry of the rotating drum 16 and the angular compartments 32 can be known, at least in terms of number and angular amplitude with respect to a round angle or 360° of rotation of the rotating drum 16, once a relation of angular position is defined between one angular compartment 32 and the rotating drum 16, the relation of angular position between each of the other remaining angular compartments 32 and the rotating drum 16 with respect to the round angle can also be derived and hence defined. For example, once the reference or "zero" position of the rotating drum 16 with respect to one individual angular compartment 32 relating to the at least one functional loading 20, 22 and/or unloading 26 aperture has been defined, then also the reference or "zero" position of the rotating drum 16 with respect to each other individual angular compartment 32 relating to the at least one functional loading 20, 22 and/or unloading 26 aperture can also be defined.

According to some embodiments, the machine 10 can include an automatic angular detection unit 15 configured to supply a reference signal relating to the angular reference position of each angular compartment 32 of the rotating drum 16 in relation to the corresponding reference or "zero" position of the rotating drum 16.

In possible variant embodiments, the automatic angular detection unit 15 can be configured to supply a reference signal relating to an angular position of a predefined angular compartment 32 of the rotating drum 16 in relation to the position of the functional loading and/or unloading aperture, which angular position of a predefined angular compartment 32 of the rotating drum 16 is associated with the predefined reference or "zero" position of the rotating drum 16.

According to some embodiments, the machine 10 can include a control unit 98 configured to
i) communicate with the automatic angular detection unit 15;
ii) select a reference position from one of the corresponding reference positions of the rotating drum 16;
iii) make the rotating drum 16 rotate to search for the selected reference or "zero" position of the rotating drum 16;
iv) dispose and maintain the rotating drum 16 in the selected reference position.

In possible variant embodiments, the control unit 98 can be configured to communicate with the automatic angular detection unit 15 and to command the drum actuation element 85, so as to make the rotating drum 16 rotate in order to search for and reach the predefined "zero" position of the rotating drum 16.

According to some embodiments, the rotation of the rotating drum 16 to search for, reach and maintain the selected or predefined "zero" position can be continued until the control unit 98 receives, from the automatic angular detection unit 15, the reference signal relating to the angular position of a predefined angular compartment 32 of the rotating drum 16 in relation to the position of the loading and/or unloading aperture, so as to make the rotating drum 16 assume the reference or "zero" position.

According to possible embodiments, the automatic angular detection unit 15 can include a sensor device 84.

Examples of sensor device 84 can be proximity or presence sensors. Possible proximity or presence sensors can be for example of the inductive, capacitive, magnetic, ultrasound or optical type. For example, a proximity or presence sensor of the optical type can be used, such as a photoelectric sensor or photocell. A sensor for photographic/video filming can also be used (camera or video camera). In possible implementations, the sensor device 84 is the magnetic type.

In possible embodiments, the sensor device 84 comprises at least one signal trigger element 86, mounted on the rotating drum 16 and disposed near the predefined angular compartment 32 of the rotating drum 16 and a sensor element 88, disposed fixed near the functional loading and/or unloading aperture and able to cooperate with the signal trigger element 86.

The sensor device 84 is configured to supply a reference signal, in particular a proximity signal, for example an alignment signal, as a function of the reciprocal position of the signal trigger element 86 and the sensor element 88. Advantageously, the control unit 98 is configured to receive the proximity signal, in order to recognize and restore the "zero" position of the rotating drum 16.

In the possible implementations where the sensor device 84 is the magnetic type, the signal trigger element 86 is a magnet, while the sensor element 88 is a magnetic field detector.

According to possible embodiments, the automatic angular detection unit 15 can also include a position transducer 89 associated with the drum actuation element 85 and configured to determine the angular position of the rotating drum 16 around the axis of rotation X. The position transducer 89 can be useful, for example, to control and command precisely the rotation of the rotating drum 16 for example when it is made to rotate step-wise, that is, intermittently, or in any case to provide the angular position of the rotating drum 16 always precisely.

According to possible embodiments, the control unit 98 is configured both to receive from the position transducer 89 the angular position signal of the rotating drum 16 around the axis of rotation X, and also to receive the reference signal from the sensor device 84. Knowing the angular position of the rotating drum 16 around the axis of rotation X before searching for the "zero" position can be advantageous in the embodiments described here, so that, when the rotating drum 16 is made to rotate by the drum actuation element 85 to search for, reach and maintain the "zero" position until the reference signal is received, the rotation imparted to the rotating drum 16 around the axis of rotation X is as brief and economical as possible. This rotation of the rotating drum 16 to search for, reach and maintain the "zero" position can be carried out, using the drum actuation element 85, with a step-wise rotation of the rotating drum 16, or with a continuous rotation. Once the "zero" position is reached, it can be maintained for example as a necessary condition for the start of a new washing and/or sterilizing cycle.

We must point out here that, by the expression "zero" position, we mean a particular reference condition recognized by the control unit 98 which for example can be decided during the construction and/or programming step of the machine 10. In particular, this condition can be identified by the alignment of the signal trigger element 86 with the sensor element 88 and set as a zero shift position for the position transducer 89.

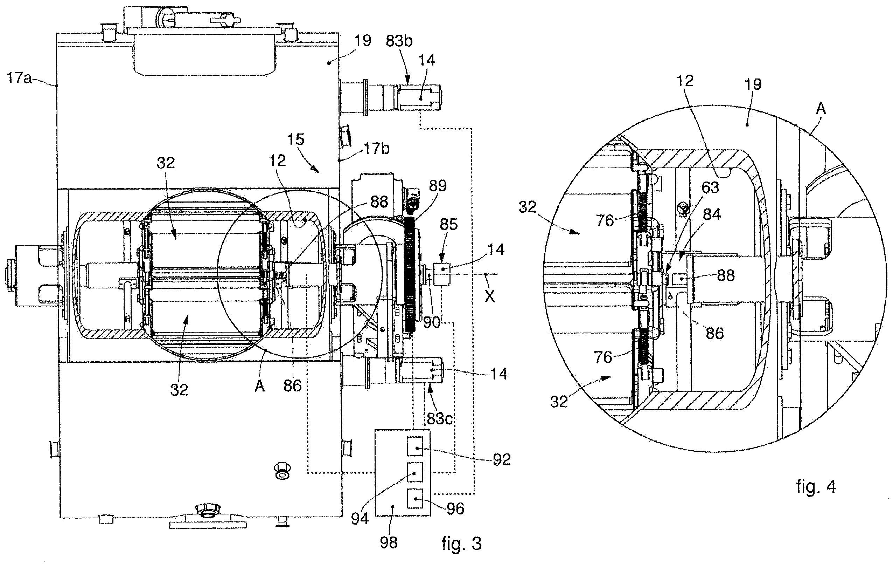

According to other embodiments, described using figs. 3-6 and combinable with all the embodiments described here, a method is provided for washing and/or sterilizing loose products which includes at least:
- loading the angular compartments 32 of the rotating drum 16 through a functional loading aperture 20, 22, for example for loading loose products to be washed and/or sterilized from the front or from the top, manually or automatically;
- activating the rotation of the rotating drum 16, proceeding to the washing and/or sterilizing of the loose products contained in the respective angular compartments 32 of the rotating drum 16;
- searching for, reaching and maintaining a predefined "zero" position of the rotating drum 16 by means of the automatic angular detection unit 15 that supplies a reference signal to the control unit 98 relating to an angular position of a predefined angular compartment 32 of the rotating drum 16 in relation to the position of a functional loading 20, 22 and/or unloading 26 aperture;
- unloading the washed and/or sterilized loose products from the angular compartments 32 of the rotating drum 16 through the functional unloading aperture 26, making the rotating drum 16 rotate step-wise with sequential angular rotation steps correlated to the angular amplitude of the angular compartments 32.

In possible implementations, the operation to search for, reach and maintain the "zero" position of the rotating drum 16 can be carried out by making the rotating drum 16 rotate continuously, or step-wise, that is, intermittently.

Possibly, after the step-wise sequential rotation to unload the various angular compartments 32, a new operation may be provided to search for, reach and maintain the "zero" position of the rotating drum 16 as described above. This can be useful, for example, if there is a partial loading or unloading of the angular compartments 32 or if any imprecision is foreseen in the complete repositioning of the rotating drum 16.

We wish to clarify here that the machine 10, for ease of exposition, in figs. 1-6 is shown without its external casing which conventionally encloses, supports and protects the internal components.

According to some embodiments described here, the machine 10 also comprises, as well as the treatment chamber 12 in which the loose products are washed and/or sterilized, a system of auxiliary plants (not shown in the drawings) cooperating with the treatment chamber 12 to create therein the necessary process conditions for washing and/or sterilizing.

The process conditions can provide specific conditions of heat or heat-moisture, obtained for example by administering in the treatment chamber 12 heat and/or steam at a desired temperature, chemical conditions, obtained for example by inserting into the treatment chamber 12 a determinate quantity of chemical agents functional for washing and/or sterilizing, pressure conditions, for example by subjecting the treatment chamber 12 to a vacuum.

According to variant embodiments, the treatment of the loose products can also include using a mixture of hot water and silicone in order to protect them.

According to possible embodiments, the treatment chamber 12 can be delimited for example by two lateral walls 17a, 17b, for example circular in shape, and by a central body or shell 19, substantially tubular in shape and which together form, for example, a cylindrical casing.

In advantageous embodiments, the drum actuation element 85 is configured to vary its speed of rotation and if necessary also to invert the direction of travel of the rotating drum 16. As explained in more detail hereafter, the drum actuation element 85 can also be operatively associated with the position transducer 89.

In possible implementations, the drum actuation element 85 as used in the embodiments described here can include a drive member 14 provided with a drive shaft 90, to make the rotating drum 16 rotate around the axis of rotation X. The drum actuation element 85 is made to function by an energy source, for example an electric current, a hydraulic fluid pressure or a pneumatic pressure.

A drive member 14 as used in association with the embodiments described here can be a drive member chosen from a group comprising: a pneumatic motor, a hydraulic piston, a piezoelectric actuator, an electric motor, a step electric motor, a magnetic motor, a linear axle with a motor, a linear motor, such as a mechanical linear motor, a piezoelectric linear motor, an electromagnetic linear motor, an electromechanical motor, an electromagnet, a gear motor, in particular a direct current gear motor. One example of a drive member 14 usable here can advantageously be a step motor that is configured to also provide a continuous rotation of the rotating drum 16. This can be useful both when it is necessary to perform a step-wise rotation of the rotating drum 16, for example for an operation to load or unload loose products, and also when it is necessary to make the rotating drum 16 rotate continuously, for example during the washing and/or sterilizing cycle.

A drum actuation element 85 as used in association with the embodiments described here can be an actuator with an intrinsically linear movement or be configured to convert a linear movement into a circular movement. The conversion can be commonly done by means of types of mechanism selected from a group consisting of: screw actuators, such as a jack screw, ball screw actuators and roll screw actuators, or wheel and axle, for example drum, gears, pulley or shaft actuators, such as a lifting cable, a winch, a rack and a pinion group, a chain transmission, a belt transmission, actuators with a rigid chain and a rigid belt.

In possible implementations, the functional loading and/or unloading apertures of the loose products can provide a manual loading aperture 20 and/or an automatic loading aperture 22, which can be alternative or both present.

In possible implementations, moreover, the functional apertures 20, 22, 26 can provide at least one unloading aperture 26.

For example, it may be provided that the unloading aperture 26 is on the opposite side of the machine 10 from the manual loading aperture 20, or in other variant embodiments, it may be provided that the unloading aperture 26 is on the same side as the manual loading aperture 20.

Furthermore, if provided, the automatic loading aperture 22 can be disposed for example on the top of the machine 10.

In possible implementations, the washing and/or sterilizing machine 10 can include a loading zone 18, for loading the loose products into the rotating drum 16, and an unloading zone 24 for the loose products after washing and/or sterilizing, for example positioned on the opposite side of the washing and/or sterilizing machine 10 with respect to the loading zone 18.

According to some embodiments, the unloading zone 24 can be a sterile chamber, or "clean room", with a controlled atmosphere.

It may be provided that the loading zone 18 is located in a front zone of the washing and/or sterilizing machine 10 and that the unloading zone 24 is located instead in a rear zone of the washing and/or sterilizing machine 10.

The loading zone 18 of the washing and/or sterilizing machine 10 can provide the manual loading aperture 20, which for example can be provided with a loading door 28, to load the loose products to be washed and/or sterilized in the treatment chamber 12.

The loading zone 18 of the washing and/or sterilizing machine 10, in possible embodiments, can also include the automatic loading aperture 22, in addition or as an alternative to the manual loading aperture 20.

The unloading zone 24 of the washing and/or sterilizing machine 10 can also include one or more unloading apertures 26, to unload the washed and/or sterilized loose products at the end of the cycle.

According to some variant embodiments, described by way of example with reference to figs. 1 and 2, the washing and/or sterilizing machine 10 can include, in its system of auxiliary plants, an automatic loading apparatus 30 to automatically feed the loose products into the rotating drum 16 inside the treatment chamber 12.

The automatic loading apparatus 30 can include, for example, a suction device able to pick up loose products in metered quantities thanks to the suction effect it creates.

In possible embodiments, the automatic loading apparatus 30 can be located in an upper zone of the treatment chamber 12, so as to feed the rotating drum 16 through the automatic loading aperture 22, pouring the loose products therein due to the effect of gravity.

In some embodiments, the rotating drum 16 has a substantially cylindrical shape and is divided into a plurality of the angular compartments 32 which can go from four to twenty-four, and which in fig. 2 are shown by way of example as twenty, shown by lines of dashes.

Again with reference to possible embodiments described using fig. 2, the rotating drum 16 comprises a support frame 34 to which all the angular compartments 32 are attached, according to a homogeneous and substantially equi-distanced circumferential distribution.

In some embodiments, the support frame 34 can comprise a hub 36, mounted solid on the drive shaft 90 to transmit the motion of the latter to the rotating drum 16.

The support frame 34 can also include a plurality of support arms 38, having one end attached to the hub 36 and branching off radially therefrom in order to connect, with its opposite end, to the angular compartments 32.

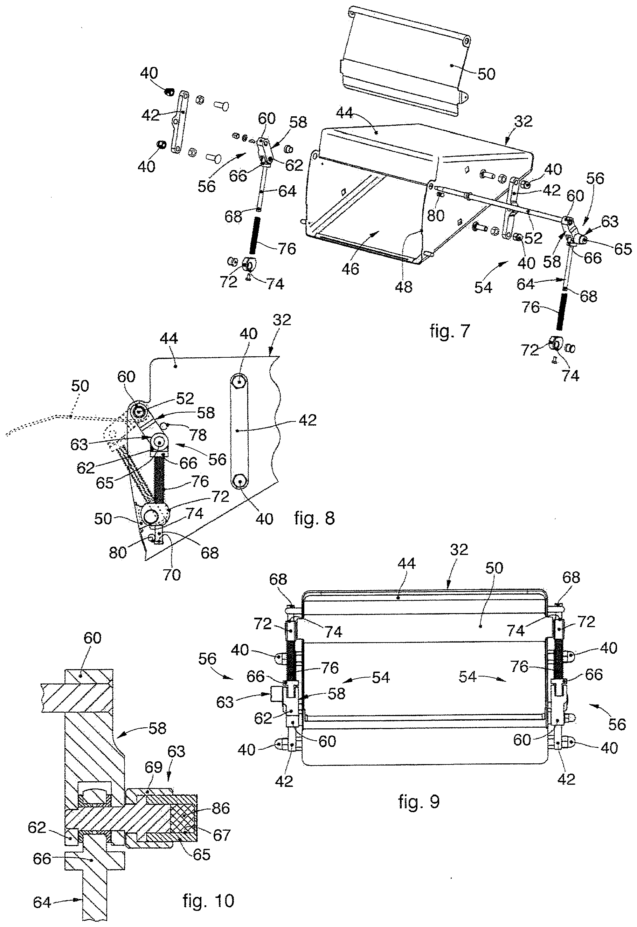

As described for example with reference to figs. 2 and 7, each angular compartment 32 is attached to the support frame 34 by removable connection means, such as for example bolts 40 and attachment brackets 42 provided to connect the angular compartments 32 directly to the support arms 38 or to the external part of the support frame 34.

Each angular compartment 32 includes a containing body 44, or tub, which can have, for example, a prismatic shape with a triangular, quadrangular, pyramidal, truncated pyramid or conical or truncated cone shape, and externally delimits a loading compartment 46. The volume of the loading compartment 46 identifies the maximum metered quantity of loose products that can be contained in each angular compartment 32.

According to possible embodiments, in the washing and/or sterilizing machine 10, the control unit 98 can be connected to the drum actuation element 85 relating to the drive shaft 90 to command the rotation of the rotating drum 16 and to position each of the angular compartments 32 in sequence selectively near the manual loading aperture 20, near the automatic loading aperture 22, or near the unloading aperture 26.

In possible implementations, the control unit 98 can be connected both to the automatic loading apparatus 30 and also to the drum actuation element 85 relating to the drive shaft 90, to control and command, in a suitably synchronized manner, the functioning of said components of the washing and/or sterilizing machine 10 in order to perform, automatically, the controlled and metered loading of the loose products into each loading compartment 46 of the angular compartments 32 of the rotating drum 16.

The division of the rotating drum 16 into angular compartments 32 is functional to the possible packaging of the loose products at the end of the washing and/or sterilizing: in fact, in this way the loose products are already divided and metered in the desired quantities for packaging.

Figs. 7-9 are used to describe possible embodiments of an angular compartment 32 with a containing body 44 with a substantially prismatic shape with a triangular base, where in correspondence with the face relating to the base size of the triangle, a loading door 48 is provided, which puts the loading compartment 46 into communication with the outside of the angular compartment 32.

Advantageously, each angular compartment 32 includes a window 50 hinged to the containing body 44, for example by a pin 52, and rotatable with respect to the latter in order to close, and keep closed, the loading door 48 during the washing and/or sterilizing treatment, and to keep the loading door 48 open during the loading and unloading of the loose products.

During use, that is, once attached to the support frame 34, the angular compartments 32 are disposed radially with respect to the drive shaft 90 and so that the loading door 48 is located in the radially most peripheral zone of the rotating drum 16.

Furthermore, each angular compartment 32 can include an opening/closing device 54 connected to the window 50 to move it from the closed position to the open position and vice versa, with respect to the loading door 48.

In possible solutions, described by way of example in figs. 7-9, the opening/closing device 54 can be symmetrical with respect to the center line of the containing body 44, and comprise two opening/closing units 56 disposed on opposite sides of the loading door 48.

The opening/closing units 56 are both connected to the window 50 to make it selectively assume the closed position, for example shown in fig. 9, in which the window 50 covers the loading door 48, and the open position, shown by lines of dashes in fig. 8, in which the open window 50 frees the loading door 48.

In some embodiments, each opening/closing unit 56 can include a crank 58 with a first end 60 connected to the hinging pin 52 of the window 50 and rotatable around the axis of the pin 52.

At least one of the two cranks 58 can also have a contact end 63, usable to open the window 50, which can be, for example, an extension of the crank 58.

The contact end 63 can also include the signal trigger element 86, as described using figs. 4, 6 and 10. Advantageously, the signal trigger element 86 can be incorporated, integrated or connected with the contact end 63. For example, the contact end 63 can be provided with an attachment portion 69, with which the signal trigger element 86 can be associated.

For example, in possible implementations, a protected housing seating 67 can be defined, associated with the contact end 63, to house and protect the signal trigger element 86.

In particular, possible implementations described using fig. 10 can provide that the contact end 63 is provided with a cover element or hood 65, which is hollow and defines the housing seating 67 to house inside it the signal trigger element 86 in a protected manner.

According to variant embodiments, the cover element 65 can be welded to the attachment portion 69, totally enclosing the signal trigger element 86 inside the housing seating 67, preventing it from coming into contact with the space where treatment takes place.

The cover element 65 is made for example of steel, which is a material suitable for health applications, that is, compatible with entering into contact with the process space of the machine 10, whereas, for example, the signal trigger element 86 could be made of a non-compatible material, and hence need protection, as in the case of the magnetic variant.

The connection between the first end 60 of the crank 58 and the pin 52 is such that with every rotation of the crank 58 there is a corresponding equal rotation of the pin 52 and consequently of the window 50 hinged to it, so that the rotation in one direction or the other of the crank 58 causes the window 50 to open or close.

The crank 58 can include a second end 62, or command end, opposite the first end 60, a rod 64 is connected with the second end 62 in correspondence with a first terminal portion 66 thereof.

The opening/closing unit 56 can include a guide bushing 72, mounted rotatable with respect to the containing body 44 with respect to an axis of rotation parallel to the axis of the pin 52, and distanced from the first end 60 of the crank 58 along a lateral delimitation wall of the loading door 48.

The rod 64 has a second terminal portion 68, opposite the first terminal portion 66, inserted transversely in the guide bushing 72, through and sliding inside it along its longitudinal extension.

The contact between the second terminal portion 68 and the guide bushing 72 allows the rod 64 to transmit the motion of the crank 58 to the guide bushing 72.

The opening/closing unit 56, in possible implementations, such as those described by way of example with reference to figs. 7-9, also includes a thrust member, such as a helical spring 76, or other similar or comparable linearly extendable element, for example a pneumatic piston, able to constantly exert a thrust force in the direction of longitudinal extension of the rod 64.

The helical spring 76 is mounted coaxial with the rod 64 and is configured to constantly exert a thrust force on both the guide bushing 72 and the second end 62 of the crank 58.

This allows to maintain the reciprocal positioning of the components of the opening/closing unit 56 both when the window 50 is in the open position, and also when it is in the closed position.

When the window 50 is closed, the helical spring 76 constantly maintains the second end 62 of the crank 58 thrust in contact with a first abutment element 78 protruding from the containing body 44, and the second terminal portion 68 of the rod 64 constantly thrust in contact with a second abutment element 80, also protruding from the containing body 44.

This prevents the spontaneous rotation of both the crank 58 and the guide bushing 72, and also the spontaneous sliding of the rod 64. To modify the positioning of the opening/closing unit 56, a moment is applied to the second end 62 of the crank 58, sufficient to overcome the thrust force of the helical spring 76. As can be seen in fig. 2, this condition can be obtained by a drive device 82a, 82b, 82c, made to rotate toward the contact end 63, obtaining an initial contact and a subsequent thrust against the latter. We must clarify here that the drive device 82a is expressly shown by a line of dashes in fig. 2, since it is optional.

With regard to the rod 64, it is provided in its second terminal portion 68 with a shoulder 70 (fig. 8), configured to limit the sliding of the rod 64 inside the guide bushing 72.

To this purpose, the guide bushing 72 includes an abutment surface 74 configured to contact the shoulder 70 of the second terminal portion 68 of the rod 64, to prevent the latter from continuing to slide with respect to the guide bushing 72 beyond what is provided in the open position of the window 50.

In possible embodiments, it may be provided that the passage of the window 50 from the closed position to the open position requires a rotation of the crank 58 of about 90° around the axis of the pin 52, to the condition of exerting a sufficient moment on the second end 62.

In the open position, the helical spring 76 maintains its thrust force against the guide bushing 72 on one side, and against the second end 62 of the crank 58 on the other side. In this way, as well as keeping the window 50 open, the helical spring 76 also has the function of keeping the shoulder 70 of the rod 64 in contact with the abutment surface 74 of the guide bushing 72.

In some embodiments, described by way of example with reference to fig. 2, the washing and/or sterilizing machine 10 can include one or more drive devices 82a, 82b, 82c associated with the treatment chamber 12 and cooperating with the opening/closing units 56 to drive them automatically or semi-automatically, and to selectively determine the passage of the windows 50 from the open position to the closed position, or vice versa.

According to variant embodiments, the rotation of the drive devices 82a, 82b, 82c can be imparted by opening/closing actuation elements 83a, 83b, 83c connected to them, of the type described previously in relation to the drum actuation element 85 connected to the drive shaft 90. We wish to clarify here that the opening/closing actuation element 83a is expressly shown by a line of dashes in fig. 1, since it is optional.

To execute the opening/closing steps, it is preferable that the opening/closing actuation elements 83a, 83b, 83c are configured to effect an inversion in the direction of travel.

The drive devices 82a, 82b, 82c can be located, non-restrictively, one near the manual loading aperture 20, one near the automatic loading aperture 22 and one near the unloading aperture 26 made in the treatment chamber 12.

The drive devices 82a, 82b, 82c can be connected to the control unit 98 and selectively activated by it in coordination with the rotation of the rotating drum 16 depending on when one of the angular compartments 32 to be loaded or unloaded is presented, on each occasion, in correspondence with one of said apertures 20, 22, 26.

The opening or closing of the angular compartments 32 can possibly be carried out selectively by an operator.

Driving the opening/closing actuation elements 83a, 83b, 83c causes the activation of the drive devices 82a, 82b, 82c, which rotate so as to contact the contact end 63 of the crank 58 of the corresponding angular compartment 32, to thus activate - opening or closing, depending on the direction of rotation of the drive device 82a, 82b, 82c - the mechanism defined by the opening/closing unit 56.

So that the window 50 stays open, the drive device 82a, 82b, 82c imparts a force to the contact end 63, impacting it, such as to overcome the resistance given by the helical spring 76. The force will be directed in one direction or the opposite direction, depending on the desired opening or closing effect.

Drive devices 82a, 82b, 82c suitable for the purposes discussed above (fig. 2) can be made by way of example in a triangular shape, with a circular, rectangular, square sector, or in any other shape suitable to obtain the purpose and also possibly of a different shape from each other due to requirements of space.

Preferably, to open or close the window 50, the alignment of the drive devices 82a, 82b, 82c and the contact end 63 of the corresponding angular compartment 32 is precise and reliable to prevent failed opening/closing and/or blockages of the rotating drum 16 of the washing and/or sterilizing machine 10.

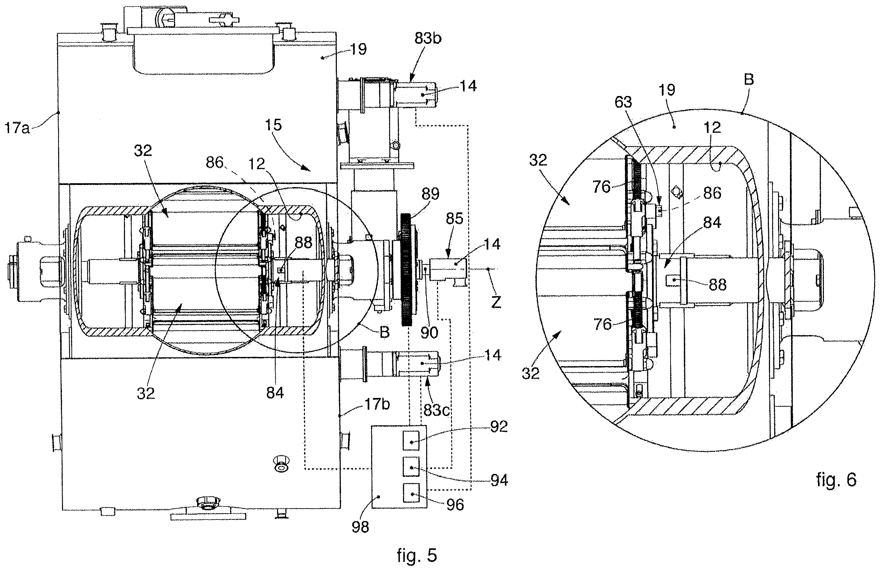

According to possible embodiments of the washing and/or sterilizing machine 10, combinable with all the embodiments described here, the sensor device 84 can be connected to communicate with the control unit 98 (figs. 3 and 5).

The signal trigger element 86 and the sensor element 88 of the sensor device 84 can be substantially cylindrical in shape and, when the "zero" position is identified, can be disposed substantially coaxial.

The signal trigger element 86 can be integrated, for example, inside the contact end 63 of a predefined containing body 44 and thus identify a reference containing body 44 or "zero tub".

The sensor element 88, instead, can be attached to the treatment chamber 12 and disposed longitudinally in a direction of an axis parallel to the axis of rotation X.

According to variant embodiments, the sensor element 88 can advantageously be located near the manual loading aperture 20, where the location can be the result of the combination of good visibility and easy access for operators, in the event of maintenance.

In possible implementations, different arrangements can also be provided with regard to the signal trigger element 86 and the sensor element 88, without departing from the field of protection of the present invention.

For example, an inverted arrangement can be provided of the signal trigger element 86 and the sensor element 88, that is, the sensor element 88 can be mounted on a reference angular compartment 32 of the rotating drum 16 and the signal trigger element 86 can be disposed fixed near the functional loading and/or unloading aperture.

When they are in the "zero" position (figs. 3 and 4), the sensor element 88 and the signal trigger element 86 are at a distance not greater than the distance that allows the sensor element 88 to detect the presence and correct disposition of the signal trigger element 86. This particular condition can be set as the zero shift position for the position transducer 89.

The position transducer 89 can be for example an angular or rotary position transducer, such as an angular or rotary encoder, in particular for example a tachometer encoder, a relative or incremental encoder, an absolute encoder. In possible implementations, the position transducer 89 can be keyed on the drive shaft 90 of the rotating drum 16. Furthermore, the position transducer 89 is advantageously configured to communicate with the control unit 98 with which it communicates to send the data relating to the angular position of the drive shaft 90, and hence of the rotating drum 16. The control unit 98 is also configured to receive from the sensor device 84 a signal correlated to the reciprocal position of the signal trigger element 86 and the sensor element 88, and this signal is correlated to the angular position of the rotating drum 16 supplied by the position transducer 89, for the purposes of realignment with the "zero" position as discussed above.

The sensor element 88 can be configured to detect the presence and correct positioning of the signal trigger element 86 associated with the reference containing body 44 or "zero tub", and supply a corresponding signal to the control unit 98.

The latter is configured to receive the signals of the sensor element 88 and consequently to command and control the movement of the drum actuation element 85 and the opening/closing actuation elements 83a, 83b, 83c, selectively activating the rotation of the rotating drum 16 and the activation of the drive devices 82a, 82b, 82c to perform the loading/unloading of the loose products.

The control unit 98 can include a central processing unit 92, or CPU, an electronic memory 94, an electronic data base 96 and auxiliary circuits (or I/O) (not shown).

For example, the CPU can be any form of computer processor used in the IT and/or automation field. The memory can be connected to the CPU and can be one or more of those commercially available, such as a random access memory (RAM), a read-only memory (ROM), floppy disk, hard disk, mass memory or any other form of digital storage, local or remote. The software instructions and data can be for example encoded and memorized in the memory to command the CPU. The auxiliary circuits can also be connected to the CPU to assist the processor in a conventional manner. The auxiliary circuits can include for example at least one of: cache circuits, feed circuits, clock circuits, input/output circuits, subsystems and suchlike. A computer-readable program (or computer instructions) can determine which tasks can be done in accordance with the method for washing and/or sterilizing loose products according to the present description. In some embodiments, the program is a computer-readable software. The computer includes a code to generate and memorize information and data introduced or generated during the course of the method according to the present description.

Figs. 3-6 are used to describe in succession the functioning of the machine for washing and/or sterilizing 10 in accordance with a method for washing and/or sterilizing loose products according to the present description, meaning that it is described in operation.

In particular, figs. 3-4 identify the condition the washing and/or sterilizing machine 10 is in when it is switched on, or immediately after. In this situation the signal trigger element 86 and the sensor element 88 are aligned as can well be seen in fig. 4, configured in the "zero" position, where, in correspondence with the manual loading aperture 20 the reference containing body 44 or "zero tub" can be found.

After the loading door 28 has been opened, the window 50 of the "zero tub" can be opened, both manually and by activating the possible opening/closing actuation element 83a relating to the drive device 82a, so that an operator can load the loose products.

Alternatively, the loading can be carried out by the automatic loading aperture 22, thanks to the automatic loading apparatus 30. In this case, the window 50 of a containing body 44, offset with respect to the "zero tub" by an integer number of containing bodies 44, is opened by activating the opening/closing actuation element 83b relating to the drive device 82b.

Subsequently, the rotating drum 16 is made to advance, advantageously step-wise, for example by an angular step correlated to the angular amplitude of the angular compartments 32, in particular with an angular amplitude substantially equal to a round angle divided by the number of angular compartments 32, in order to fill subsequent containing bodies 44 with loose products. This can be obtained by sending, by the position transducer 89, the datum of the current position to the control unit 98 which in turn activates the drum actuation element 85 to rotate.

According to variant embodiments, the containing bodies 44 can also be loaded by a cooperation of manual and automatic loading, in order to speed up this operation.

The loose products can be loaded both by totally filling the volume identified by the containing bodies 44, and by filling it partly.

Furthermore, the loading of the loose products can affect the totality of the angular compartments 32, giving rise to a total or complete loading, or it can affect only one part of the angular compartments 32, preferably contiguous, thus giving rise to a partial loading.

Once the angular compartments 32 of the rotating drum 16 have been loaded in the desired way, it is possible to start the treatment cycle of the loose products, activating the drum actuation element 85 which makes the drive shaft 90 rotate for the necessary time for washing and/or sterilizing.

When the treatment is finished, or when the control unit 98 has detected a signal, or asynchronous impulse, arriving for example from the pressure of a stop button by an operator (not shown in the drawings), the position of the reference containing body 44 or "zero tub", and hence of the respective angular compartment 32, could be anywhere. Therefore, the shift between the signal trigger element 86 and the sensor element 88 could be any whatsoever, as identified in figs. 5 and 6.

By "asynchronous impulse" we mean a signal deriving from a non-predictable or periodic event, such as an un-programmed request to interrupt the process, for example an alarm signal, which the control unit 98 is able to detect and manage.

If the washing and/or sterilizing machine 10 is stopped by the operator, or in any case to carry out the programmed unloading of the loose products at the end of the treatment, it is useful to return to the "zero" position.

Therefore, if the rotating drum 16 has stopped in any position whatsoever, the shift between the signal trigger element 86 and the sensor element 88 is identified by the position transducer 89 which makes this information available to the control unit 98. Consequently, the drum actuation element 85 is commanded by the control unit 98 and activated to rotate the drive shaft 90 until the sensor element 88 detects the presence of the signal trigger element 86. This event produces a new signal that is sent by the sensor element 88 to the control unit 98, which quickly stops the drum actuation element 85 by stopping the drive shaft 90 in the "zero" position, so that in this way the rotating drum 16 is disposed and maintained in the selected reference position.

Advantageously it may be provided that the drum actuation element 85 can activate the drive shaft 90 to rotate in one direction or the other according to the value of the shift detected by the position transducer 89, so as to effect the minimum rotation possible to realign the signal trigger element 86 with the sensor element 88.

After the rotating drum 16 has found the "zero" position, the step to unload the washed and/or sterilized loose products from the unloading aperture 26 can be started. The activation by the control unit 98 of the opening/closing actuation element 83c leads to the opening of the window 50 of a containing body 44, offset from the "zero tub" by an integer number of containing bodies 44, by rotating the drive device 82c; it then also leads to the emptying of the containing body 44, for example due to the effect of gravity.

Consequently, the rotating drum 16 is made to advance step-wise, by a step with an angular amplitude substantially equal to a round angle divided by the number of angular compartments 32, in order to empty the subsequent containing bodies 44 of the loose products, with a procedure like that described above.

The method provides to repeat the emptying at least of the containing bodies 44 that were previously filled with loose products.

Once emptying is finished, the control unit 98 again repositions the rotating drum 16 in the "zero" position in the same way as described above, as a consequence of receiving the signal sent by the sensor element 88 when it detects the presence of the signal trigger element 86, thus allowing the washing and/or sterilizing machine 10 to be able to carry out a new washing and/or sterilizing treatment of loose products.

Preferably, the control unit 98 can be configured to impart constant or variable speeds and/or accelerations to the rotating drum 16. The speeds and/or accelerations can be constant or variable, for example to adapt to the washing and/or sterilizing steps or to the various loading/unloading steps of the loose products described here.

Some embodiments can provide the execution of various steps, passages and operations, as described above. The steps, passages and operations can be done at least partly with instructions performed by a machine which cause the execution of certain steps by a general-purpose or special-purpose processor. Alternatively, these steps, passages and operations can be performed by specific hardware components that contain hardware logic to perform the steps, or by any combination of components for programmed computers and personalized hardware components.

Embodiments of the method in accordance with the present description can be included in a program for computers that can be memorized in a computer-readable mean that includes the instructions that, once performed by the washing and/or sterilizing machine 10, determine the execution of the method discussed here.

In particular, elements of the present invention can be given as machine-readable means to memorize the instructions which can be carried out by the machine. The machine-readable means can include, without being limited to, floppy disks, optical disks, CD-ROM, optical-magnetic disks, ROM, RAM, EPROM, EEPROM, optical or magnetic cards, propagation means or other types of machine-readable means suitable to memorize electronic information. For example, at least parts of the present invention can be downloaded as a computer program that can be transferred from a remote computer (for example a server) to a requesting computer (for example a client), by means of data signals received with carrier waves or other propagation means, via a communication connection (for example a modem or a network connection).

It is clear that modifications and/or additions of parts may be made to the washing and/or sterilizing machine 10 as described heretofore, without departing from the field and scope of the present invention.

It is also clear that, although the present invention has been described with reference to some specific examples, a person of skill in the art shall certainly be able to achieve many other equivalent forms of washing and/or sterilizing machine 10, having the characteristics as set forth in the claims and hence all coming within the field of protection defined thereby.

Although the above refers to forms of embodiment of the invention, other forms of embodiment can be provided without departing from the main field of protection, which is defined by the following claims.

## Claims

1. Machine for washing and/or sterilizing loose products comprising a treatment chamber (12) provided with at least one functional loading (20, 22) and/or unloading (26) aperture and containing a rotating drum (16) provided with a plurality of angular compartments (32), said machine comprising at least one drum actuation element (85), provided with a drive shaft (90), configured to make the rotating drum (16) rotate around an axis of rotation (X),
**characterized in that**
- for each of said angular compartments (32), at least a corresponding reference position of the rotating drum (16) is predefined with respect to the position of the individual compartment relative to the at least one functional loading (20, 22) and/or unloading (26) aperture;
and said machine comprises:
- at least one automatic angular detection unit (15) configured to supply a reference signal relating to the reference angular position of each angular compartment (32) of the rotating drum (16) in relation to said corresponding reference position of the rotating drum (16);
- at least one control unit (98) configured to:
i) communicate with the automatic angular detection unit (15);
ii) select a reference position from one of said corresponding reference positions of the rotating drum (16);
iii) make the rotating drum (16) rotate to search for said selected reference position;
iv) dispose and maintain the rotating drum (16) in said selected reference position.

2. Machine as in claim 1, **characterized in that** said control unit (98) is configured to maintain said predefined angular reference position of the rotating drum (16) after receiving from the automatic angular detection unit (15) the reference signal relating to the angular position of said predefined angular compartment (32) of the rotating drum (16) in relation to the position of said functional loading and/or unloading aperture.

3. Machine as in claim 1 or 2, **characterized in that** the automatic angular detection unit (15) comprises a sensor device (84) provided with at least one signal trigger element (86) and at least one sensor element (88).

4. Machine as in claim 3, wherein the sensor element (88) is positioned in proximity to said at least one functional loading and/or unloading aperture.

5. Machine as in claim 3 or 4, wherein the signal trigger element (86) is mounted on one angular compartment (32).

6. Machine as in claim 3, 4 or 5, wherein the sensor element (88) is attached to a lateral wall (17b) of the treatment chamber (12).

7. Machine as in any claim hereinbefore, wherein the automatic angular detection unit (15) comprises at least a position transducer (89) configured to detect the angular position of the rotating drum (16) around the axis of rotation (X).

8. Machine as in claim 7, wherein the position transducer (89) is keyed on the drive shaft (90) of the rotating drum (16).

9. Machine as in any claim hereinbefore, wherein said at least one functional loading and/or unloading aperture comprises at least one manual loading aperture (20) and/or at least one automatic loading aperture (22) for loading loose products and/or at least one unloading aperture (26) of the loose products.

10. Machine as in claim 9, wherein a drive device (82a, 82b, 82c) is provided, associated with the at least one manual loading aperture (20) and/or with the at least one automatic loading aperture (22) and/or with the at least one unloading aperture (26) and configured to selectively open and close the angular compartments (32) of the rotating drum (16).

11. Method for washing and/or sterilizing loose products using a machine (10) as in any of the previous claims from 1 to 10, **characterized in that** said method comprises:
- loading angular compartments (32) of a rotating drum (16) through a manual functional loading aperture (20) and/or an automatic loading aperture (22), with loose products to be washed and/or sterilized;
- activating the rotation of the rotating drum (16), proceeding to the washing and/or sterilizing of the loose products contained in respective angular compartments (32) of the rotating drum (16);
- searching for and maintaining a predefined angular reference or "zero" position, of the rotating drum (16) by means of an automatic angular detection unit (15) that supplies a reference signal to a control unit (98) relating to an angular position of a predefined angular compartment (32) of the rotating drum (16) in relation to the position of the manual functional loading aperture (20) and/or the automatic loading aperture (22) and/or an unloading aperture (26) of the loose products;
- unloading the washed and/or sterilized loose products from the angular compartments (32) of the rotating drum (16) through the unloading aperture (26), making the rotating drum (16) rotate step-wise with sequential angular rotation steps correlated to the angular amplitude of the angular compartments (32).

12. Method as in claim 11, wherein the control unit (98) activates a drum actuation element (85) making a drive shaft (90) rotate in one direction or the other on the basis of an angular shift value detected by a position transducer (89) associated with the drum actuation element (85) with respect to the predefined angular reference position of the rotating drum (16).

13. Method as in claim 11 or 12, wherein after the sequential step-wise rotation to unload the various angular compartments (32), it provides to make a new operation to search for and maintain the "zero" position of the rotating drum (16).

14. Computer program storable in a non-transitory data support readable by a computer that contains the instructions that, once carried out by a machine (10) as in any of the claims from 1 to 10, determine the execution of the method as in any of claims 11, 12 or 13.

## Patentansprüche

1. Vorrichtung zum Waschen und/oder Sterilisieren von losen Produkten, aufweisend eine Behandlungskammer (12), die mit mindestens einer funktionellen Belade- (20, 22) und/oder Entlade-Öffnung (26) versehen ist und eine rotierende Trommel (16) enthält, die mit einer Mehrzahl von winkligen Kammern (32) versehen ist, wobei die Vorrichtung mindestens ein mit einer Antriebswelle (90) versehenes Trommel-Betätigungs-Element (85) aufweist, das konfiguriert ist, um zu bewirken, dass sich die rotierende Trommel (16) um eine Rotationsachse (X) dreht,
**dadurch gekennzeichnet, dass**
- für jede der winkligen Kammern (32) mindestens eine entsprechende Referenzposition der rotierenden Trommel (16) bezüglich der Position der einzelnen Kammer relativ zu der mindestens einen funktionellen Belade- (20, 22) und/oder Entlade-Öffnung (26) vordefiniert ist,
und die Vorrichtung aufweist:
- mindestens eine automatische Winkeldetektionseinheit (15), die konfiguriert ist, um ein Referenzsignal, das die Referenz-Winkelposition jeder winkligen Kammer (32) der rotierenden Trommel (16) betrifft, bezüglich der entsprechenden Referenzposition der rotierenden Trommel (16) bereitzustellen,
- mindestens eine Steuereinheit (98), die konfiguriert ist, um:
i) mit der automatischen Winkeldetektionseinheit (15) zu kommunizieren,
ii) eine Referenzposition aus einer der entsprechenden Referenzpositionen der rotierenden Trommel (16) auszuwählen,
iii) zu bewirken, dass sich die rotierende Trommel (16) dreht, um die ausgewählte Referenzposition zu suchen,
iv) die rotierende Trommel (16) in der ausgewählten Referenzposition anzuordnen und zu halten.

2. Vorrichtung gemäß Anspruch 1, **dadurch gekennzeichnet, dass** die Steuereinheit (98) konfiguriert ist, um die vordefinierte Winkelreferenzposition der rotierenden Trommel (16) nach dem Empfangen des die Winkelposition der vordefinierten winkligen Kammer (32) der rotierenden Trommel (16) betreffenden Referenzsignals von der automatischen Winkeldetektionseinheit (15) in Bezug auf die Position der funktionellen Belade- und/oder Entlade-Öffnung zu halten.

3. Vorrichtung gemäß Anspruch 1 oder 2, **dadurch gekennzeichnet, dass** die automatische Winkeldetektionseinheit (15) eine Sensorvorrichtung (84) aufweist, die mit mindestens einem Signal-Auslöse-Element (86) und mindestens einem Sensorelement (88) versehen ist.

4. Vorrichtung gemäß Anspruch 3, wobei das Sensorelement (88) in der Nähe der mindestens einen funktionellen Belade- und/oder Entlade-Öffnung positioniert ist.

5. Vorrichtung gemäß Anspruch 3 oder 4, wobei das Signal-Auslöse-Element (86) an einer winkligen Kammer (32) montiert ist.

6. Vorrichtung gemäß Anspruch 3, 4 oder 5, wobei das Sensorelement (88) an einer seitlichen Wand (17b) der Behandlungskammer (12) angebracht ist.

7. Vorrichtung gemäß irgendeinem vorhergehenden Anspruch, wobei die automatische Winkeldetektionseinheit (15) mindestens einen Positionsgeber (89) aufweist, der konfiguriert ist, um die Winkelposition der rotierenden Trommel (16) um die Rotationsachse (X) zu detektieren.

8. Vorrichtung gemäß Anspruch 7, wobei der Positionsgeber (89) auf die Antriebswelle (90) der rotierenden Trommel (16) aufgekeilt ist.

9. Vorrichtung gemäß irgendeinem vorhergehenden Anspruch, wobei die mindestens eine funktionelle Belade- und/oder Entlade-Öffnung mindestens eine Manuell-Belade-Öffnung (20) und/oder mindestens eine Automatik-Belade-Öffnung (22) zum Laden von losen Produkten und/oder mindestens eine Entlade-Öffnung (26) für die losen Produkte aufweist.

10. Vorrichtung gemäß Anspruch 9, wobei eine Antriebsvorrichtung (82a, 82b, 82c) vorgesehen ist, die mit der mindestens einen Manuell-Belade-Öffnung (20) und/oder mit der mindestens einen Automatik-Belade-Öffnung (22) und/ oder mit der mindestens einen Entlade-Öffnung (26) verknüpft ist und konfiguriert ist, um die winkligen Kammern (32) der rotierenden Trommel (16) wahlweise zu öffnen und zu schließen.

11. Verfahren zum Waschen und/oder Sterilisieren von losen Produkten mittels einer Vorrichtung (10) gemäß irgendeinem der vorhergehenden Ansprüche 1 bis 10, **dadurch gekennzeichnet, dass** das Verfahren aufweist:
- Beladen von winkligen Kammern (32) einer rotierenden Trommel (16) durch eine funktionelle Manuell-Belade-Öffnung (20) und/oder eine Automatik-Belade-Öffnung (22) mit zu waschenden und/oder zu sterilisierenden losen Produkten,
- Aktivieren der Rotation der rotierenden Trommel (16), Durchführen des Waschens und/oder Sterilisierens der losen Produkte, die in jeweiligen winkligen Kammern (32) der rotierenden Trommel (16) enthalten sind,
- Suchen und Halten einer vordefinierten Winkelreferenz- oder "Null"-Position der rotierenden Trommel (16) mittels einer automatischen Winkeldetektionseinheit (15), die einer Steuereinheit (98) ein Referenzsignal zuführt, das eine Winkelposition einer vordefinierten winkligen Kammer (32) der rotierenden Trommel (16) betrifft, mit Bezug auf die Position der funktionellen Manuell-Belade-Öffnung (20) und/oder der Automatik-Belade-Öffnung (22) und/oder einer Entlade-Öffnung (26) für die losen Produkte,
- Entladen der gewaschenen und/oder sterilisierten losen Produkte aus den winkligen Kammern (32) der rotierenden Trommel (16) durch die Entlade-Öffnung (26), wobei bewirkt wird, dass sich die rotierende Trommel (16) schrittweise in sequentiellen Winkelrotationsschritten dreht, die mit der Winkelamplitude der winkligen Kammern (32) korrelieren.

12. Verfahren gemäß Anspruch 11, wobei die Steuereinheit (98) ein Trommel-Betätigungs-Element (85) aktiviert, das bewirkt, dass sich eine Antriebswelle (90) in der einen oder der anderen Richtung dreht, auf der Grundlage eines Winkelverschiebungswertes, der von einem Positionsgeber (89) detektiert wird, der mit dem Trommel-Betätigungs-Element (85) verknüpft ist, mit Bezug auf die vordefinierte Winkelreferenzposition der rotierenden Trommel (16).

13. Verfahren gemäß Anspruch 11 oder 12, wobei es nach dem sequentiellen schrittweisen Drehen zum Entladen der verschiedenen winkligen Kammern (32) vorsieht, einen neuen Vorgang durchzuführen, um die "Null"-Position der rotierenden Trommel (16) zu suchen und zu halten.

14. Computerprogramm, das in einem dauerhaften Datenträger speicherbar ist, der mittels eines Rechners lesbar ist, und das die Befehle enthält, die beim Durchführen durch eine Vorrichtung (10) gemäß irgendeinem der Ansprüche 1 bis 10 die Durchführung des Verfahrens gemäß irgendeinem der Ansprüche 11, 12 oder 13 veranlassen.

## Revendications

1. Machine pour le lavage et/ou la stérilisation de produits en vrac, comprenant qui une chambre de traitement (12) possédant au moins une ouverture fonctionnelle de chargement (20, 22) et/ou de déchargement (26) et contenant un tambour rotatif (16) possédant une pluralité de compartiments angulaires (32), ladite machine comprenant au moins un élément d'actionnement (85) du tambour, pourvu d'un arbre d'actionnement (90), conçu pour faire tourner le tambour rotatif (16) autour d'un axe de rotation (X),
**caractérisée en ce que**
- pour chacun desdits compartiments angulaires (32), au moins une position de référence du tambour rotatif (16), relative à la position du compartiment individuel, est définie par rapport à l'au moins une ouverture fonctionnelle de chargement (20, 22) et/ou de déchargement (26) ;
et ladite machine comprend :
- au moins une unité de détection angulaire automatique (15) conçue pour fournir un signal de référence relatif à la positon angulaire de référence de chaque compartiment angulaire (32) du tambour rotatif (16) par rapport à ladite position de référence correspondante du tambour rotatif (16) ;
- au moins une unité de commande (98) conçue pour :
i) communiquer avec l'unité de détection angulaire automatique (15) ;
ii) sélectionner une position de référence comme l'une desdites positions de référence correspondantes du tambour rotatif (16) ;
iii) faire tourner le tambour rotatif (16) pour chercher ladite position de référence sélectionnée ;
iv) placer et maintenir le tambour rotatif (16) dans ladite position de référence sélectionnée.

2. Machine selon la revendication 1, **caractérisée en ce que** ladite unité de commande (98) est conçue pour maintenir ladite position angulaire de référence prédéfinie du tambour rotatif (16) après avoir reçu de l'unité de détection angulaire automatique (15) le signal de référence relatif à la position angulaire dudit compartiment angulaire prédéfini (32) du tambour rotatif (16) par rapport à la position de ladite ouverture fonctionnelle de chargement et/ou de déchargement.

3. Machine selon la revendication 1 ou 2, **caractérisée en ce que** l'unité de détection angulaire automatique (15) comprend un dispositif capteur (84) pourvu d'au moins un élément de déclenchement de signal (86) et d'au moins un élément capteur (88).

4. Machine selon la revendication 3, dans laquelle l'élément capteur (88) est positionné à proximité de ladite au moins une ouverture fonctionnelle de chargement et/ou de déchargement.

5. Machine selon la revendication 3 ou 4, dans laquelle l'élément de déclenchement de signal (86) est monté sur un compartiment angulaire (32).

6. Machine selon la revendication 3, 4 ou 5, dans lequel l'élément capteur (88) est attaché à une paroi latérale (17b) de la chambre de traitement (12).

7. Machine selon n'importe laquelle des revendications précédentes, dans laquelle l'unité de détection angulaire automatique (15) comprend au moins un transducteur de position (89) conçu pour détecter la position angulaire du tambour rotatif (16) autour de l'axe de rotation (X).

8. Machine selon la revendication 7, dans laquelle le capteur de position (89) est calé sur l'arbre d'entraînement (90) du tambour rotatif (16).

9. Machine selon n'importe laquelle des revendications précédentes, dans laquelle ladite au moins une ouverture fonctionnelle de chargement et/ou de déchargement comprend au moins une ouverture de chargement manuel (20) et/ou au moins une ouverture de chargement automatique (22) pour le chargement de produits en vrac et/ou au moins une ouverture de déchargement (26) des produits en vrac.

10. Machine selon la revendication 9, comportant un dispositif d'entraînement (82a, 82b, 82c), associé à l'au moins une ouverture de chargement manuel (20) et/ou à l'au moins une ouverture de chargement automatique (22) et/ou à l'au moins une ouverture de déchargement (26) et conçu pour ouvrir et fermer sélectivement les compartiments angulaires (32) du tambour rotatif (16).

11. Procédé de lavage et/ou de stérilisation de produits en vrac à l'aide d'une machine (10) selon n'importe laquelle des revendications 1 à 10, **caractérisé en ce que** ledit procédé comprend les étapes consistant à :
- charger des produits en vrac à laver et/ou stériliser dans des compartiments angulaires de chargement (32) d'un tambour rotatif (16) à travers une ouverture fonctionnelle de chargement manuel (20) et/ou une ouverture de chargement automatique (22) ;
- activer la rotation du tambour rotatif (16), et effectuer le lavage et/ou la stérilisation des produits en vrac contenus dans des compartiments angulaires respectifs (32) du tambour rotatif (16) ;
- chercher et maintenir une référence angulaire prédéfinie ou une position « zéro » du tambour rotatif (16), au moyen d'une unité de détection angulaire automatique (15) qui fournit un signal de référence à une unité de commande (98) en relation à une position angulaire d'un compartiment angulaire prédéfini (32) du tambour rotatif (16) par rapport à la position de l'ouverture fonctionnelle de chargement manuel (20) et/ou de l'ouverture de chargement automatique (22) et/ou d'une ouverture de déchargement (26) des produits en vrac;
- décharger les produits en vrac lavés et/ou stérilisés des compartiments angulaires (32) du tambour rotatif (16) à travers l'ouverture de déchargement (26), par une rotation pas-à-pas du tambour rotatif (16) avec des pas de rotation angulaires consécutifs corrélés à l'amplitude angulaire des compartiments angulaires (32).

12. Procédé selon la revendication 11, dans lequel l'unité de commande (98) active un élément d'actionnement du tambour (85), et fait tourner un arbre d'entraînement (90) dans un sens ou dans l'autre sur la base d'une valeur de décalage angulaire détectée par un transducteur de position (89) associé à l'élément d'actionnement (85) du tambour par rapport à la position de référence angulaire prédéfinie du tambour rotatif (16).

13. Procédé selon la revendication 11 ou 12, dans lequel, après la rotation par pas consécutifs pour décharger les compartiments angulaires différents (32), une nouvelle opération est effectuée pour chercher et maintenir la position « zéro » du tambour rotatif (16).

14. Programme informatique pouvant être mémorisé sur un support de données non-temporaire lisible par ordinateur, contenant les instructions qui, une fois qu'elles sont exécutées par une machine (10) selon n'importe laquelle des revendications 1 à 10, comportent l'exécution du procédé selon n'importe laquelle des revendications 11, 12 ou 13.
